(19) Europäisches Patentamt
European Patent Office
Office européen des brevets

(11) **EP 3 977 107 B1**

(12) **EUROPEAN PATENT SPECIFICATION**

(45) Date of publication and mention
of the grant of the patent:
**15.07.2026 Bulletin 2026/29**

(21) Application number: **19733253.9**

(22) Date of filing: **31.05.2019**

(51) International Patent Classification (IPC):
*G01N 21/05* (2006.01)     *G01N 21/82* (2006.01)
*G01N 33/49* (2006.01)     *G01N 15/14* (2024.01)
*G01N 15/1433* (2024.01)   *G01N 15/0227* (2024.01)
*B01L 3/00* (2006.01)      *G01N 15/00* (2024.01)
*G01N 15/01* (2024.01)     *G01N 15/10* (2024.01)

(52) Cooperative Patent Classification (CPC):
**G01N 21/82; B01L 3/502761; G01N 15/0227;
G01N 15/1433; G01N 15/1459; G01N 21/05;
G01N 33/4915;** B01L 2200/0652; B01L 2300/0877;
G01N 2015/0092; G01N 2015/018;
G01N 2015/1006; G01N 2015/1027;
G01N 2015/1493; G01N 2021/825

(86) International application number:
**PCT/US2019/035023**

(87) International publication number:
**WO 2020/242502 (03.12.2020 Gazette 2020/49)**

(54) **IN-VITRO METHODS TO EVALUATE HEMOSTATIC AGENTS IN CAPILLARY MODELS**

IN-VITRO-VERFAHREN ZUR BEWERTUNG VON HÄMOSTATISCHEN MITTELN IN
KAPILLARMODELLEN

PROCÉDÉS D'ÉVALUATION IN VITRO D'AGENTS HÉMOSTATIQUES DANS DES MODÈLES
CAPILLAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**06.04.2022 Bulletin 2022/14**

(73) Proprietor: **Colgate-Palmolive Company
New York, NY 10022 (US)**

(72) Inventors:
• **XU, Shao Peng
Guangzhou, Guangdong 510730 (CN)**
• **CHEN, Dai Lin
Guangzhou, Huangpu 510730 (CN)**

(74) Representative: **Sonnenhauser, Thomas Martin
Wuesthoff & Wuesthoff
Patentanwälte und Rechtsanwalt PartG mbB
Schweigerstraße 2
81541 München (DE)**

(56) References cited:
**EP-B1- 3 672 564        WO-A1-2019/108215
US-A1- 2011 045 993      US-A1- 2014 255 961
US-A1- 2019 167 555**

• **MENG Q: "Anti-bacterial toothpaste comprises
anti-inflammatory hemostatic component, anti-
allergic and analgesic component and base
component", WPI / 2017 CLARIVATE
ANALYTICS,, vol. 2017, no. 35, 15 March 2017
(2017-03-15), XP002777657**

## Description

## BACKGROUND

The present invention is directed to in-vitro platelet evaluation methods. Gum bleeding is associated with many common oral conditions, such as gingivitis. Gum bleeding may be caused by a buildup of plaque, a soft, sticky, colorless film of bacteria that forms on the teeth and gums, and produces toxins that may inflame or infect the gum tissue to cause gingivitis. Gingivitis is the initial stage of gum disease and, if left untreated, may cause periodontitis.

Various clinical evaluation methods have been developed to evaluate the efficacy of oral care compositions on gum bleeding, such as the Gingival Bleeding Index, the Sulcus Bleeding Index, and the Papillary Bleeding Score, among others. However, there is a lack of pre-clinical and/or in-vitro testing to evaluate the efficacy of oral care compositions on gum bleeding or as hemostatic agents.

Accordingly, it would be useful to develop systems and methods for the pre-clinical and/or in-vitro evaluation of oral care compositions or active ingredients on gum bleeding and/or as hemostatic agents. And in particular, in-vitro system and methods to evaluate the efficacy of oral care compositions or active ingredients as hemostatic agents in capillary models. US 2011/0045993 A1 discloses An object/test material interaction microfluidic device comprising a sample inlet adapted to receive a fluid sample comprising a plurality of objects, an outlet adapted to output the fluid sample from the device, at least one internal surface defining a flow cavity within the device, wherein the flow cavity extends between and is connected to the sample inlet and the outlet for flow of the fluid sample through the flow cavity, the flow cavity comprises a test area to which at least one test material is attached and which is situated in the flow cavity for flow of the fluid sample over the test area, and the flow cavity has an aspect ratio which, when the flow cavity is substantially filled by the fluid sample, provides a substantially constant shear force between the test area and the fluid sample flowing over the test area. The abstract "Anti-bacterial toothpaste comprises anti-inflammatory hemostatic component, anti-allergic and analgesic component and base component", by Meng Q., published by Clarivate Analytics, vol. 2017, no. 35, 15 March 2017, discloses an anti-bacterial toothpaste which comprises anti-inflammatory hemostatic component, anti-allergic and analgesic component, wherein the anti-inflammatory hemostatic component comprises 0.1-2 wt.% eugenol and 0.1-2 wt.% red salt, wherein the anti-allergic and analgesic component comprises 0.1-2 wt.% paclitaxel and 0.1-1 wt.% paeonol, where paclitaxel is zanthaxanthin, wherein the base component comprises 1-22 wt.% silica, 0.2 wt.% cellulose glue, 0.2-1.0 wt.% xanthan gum, 1-60 wt.% sorbitol, 1-3 wt.% perfume, 1-4 wt.% sodium lauryl sulfate and 0.3 wt.% titanium dioxide. US 2014/0255961 A1 discloses a method of assaying wound healing can include: growing cells on the matrix in the first flow channel; introducing an agent that removes the matrix from the junction; introducing a matrix material into the second flow channel so as to form the second matrix in the second flow channel and junction; and detecting cellular migration into the junction onto the second matrix, wherein the agent that removes the matrix can include a biomolecule or chemical agent, wherein the method can include removing cells in the matrix in the junction before introducing the matrix material into the second flow channel., wherein a bioactive agent can be introduced into the junction to determine if it modulates cellular migration and/or clot formation into the intersection openings of tissue and vascular channels.

## BRIEF SUMMARY

The present invention is directed to an in-vitro platelet evaluation method as defined in appended independent claims 1 and 6. Particular embodiments of the invention are defined in appended dependent claims 2-5. The scope of the invention is solely defined and limited by the appended claims.

Further areas of applicability of the present invention will become apparent from the detailed description provided hereinafter.

The foregoing and/or other aspects and utilities embodied in the present disclosure are achieved by providing an in-vitro platelet evaluation method as defined in the claims, including, amongst other features defined in the claims, creating a platelet suspension sample; creating a test substrate, wherein the test substrate comprises collagen; setting up an evaluation system, including placing the test substrate within the evaluation system; flowing the platelet suspension sample through the evaluation system; modifying the platelet suspension sample flow through the evaluation system; stopping the platelet suspension sample flow through the evaluation system; creating a test platelet suspension; flowing the test platelet suspension through the evaluation system; modifying the test platelet suspension flow through the evaluation system; stopping the test platelet suspension flow through the evaluation system; and wherein the evaluation system comprises a vacuum pump as further defined in the claims.

The evaluation system may include a media source that holds at least one of the platelet suspension sample and the test platelet suspension, a flow chamber that receives at least one of the platelet suspension sample and the test platelet suspension from the media source, a flow pump that facilitates flow of at least one of the platelet suspension sample and the test platelet suspension through the flow chamber, a microscope that is configured to evaluate at least one of the platelet suspension sample and the test platelet suspension in the flow chamber, and a camera and a computer that are operably connected to the microscope and that are configured to record an image through the microscope and facilitate evaluation of at least one of the platelet suspension sample and the test platelet suspension in the flow chamber.

The flow chamber may be a parallel-plate flow chamber including a base plate configured to control the flow of at least one of the platelet suspension sample and the test platelet suspension through the flow chamber, a slide plate configured to receive at least one of the platelet suspension sample and the test platelet suspension from the media source, and a gasket configured to control a height of a flow path in the flow chamber, wherein the base plate includes a flow inlet configured to connect to the media source, a flow outlet configured to connect to the flow pump, and a vacuum port configured to connect to the vacuum pump.

Modifying of the platelet suspension sample flow through the evaluation system includes modifying the flow rate of the platelet suspension sample to mimic a capillary bleeding model flow through the evaluation system; and the method further includes recording a behavior of the platelets in the platelet suspension sample in the evaluation system.

The flow rate to mimic a capillary bleeding model through the evaluation system is 0.04 mL/min.

The behavior of the platelets in the platelet suspension sample may be recorded for 2 minutes at 50 fps/s.

The recording of the behavior of the platelets in the platelet suspension sample in the evaluation system is used to determine the platelet aggregation area and platelet rolling velocity for the platelet suspension sample.

Creating a test platelet suspension includes adding a sample of an oral care composition or active agent to the platelet suspension sample to create the test platelet suspension.

Modifying of the test platelet suspension flow through the evaluation system includes modifying the flow rate of the test platelet suspension to mimic a capillary bleeding model flow through the evaluation system; and the method may further include recording a behavior of the platelets in the test platelet suspension in the evaluation system.

The flow rate to mimic a capillary bleeding model through the evaluation system is 0.04 mL/min.

The behavior of the platelets in the test platelet suspension may be recorded for 2 minutes at 50 fps/s.

The recording of the behavior of the platelets in the test platelet suspension through the evaluation system is used to determine the platelet aggregation area and platelet rolling velocity for the test platelet suspension.

The foregoing and/or other aspects and utilities embodied in the present disclosure are also achieved by providing an in-vitro platelet evaluation method as defined in the claims, including, amongst other features defined in the claims, creating a platelet suspension sample; creating a test platelet suspension by adding a test agent to the platelet suspension sample; flowing the platelet suspension sample through an evaluation system and recording a behavior of platelets in the platelet suspension sample in the evaluation system; flowing the test platelet suspension through the evaluation system and recording a behavior of platelets in the test platelet suspension in the evaluation system; comparing the behavior of platelets in the platelet suspension sample in the evaluation system to the behavior of platelets in the test platelet suspension in the evaluation system to determine a hemostatic efficacy of the test agent; wherein at least one of the flowing the platelet suspension sample through the evaluation system and the flowing of the test platelet suspension through the evaluation system comprises modifying a flow rate to mimic a capillary bleeding model flow through the evaluation system, and wherein comparing the behavior of the platelets comprises comparing the average platelet aggregation area and the average platelet rolling velocity of the platelet suspension sample and the test platelet suspension.

## BRIEF DESCRIPTION OF THE DRAWINGS

The accompanying drawings, which are incorporated in, and constitute a part of this specification, illustrate implementations of the present teachings and, together with the description, serve to explain the principles of the disclosure. In the figures:

FIG. 1 illustrates an in-vitro platelet evaluation system according to an implementation of the present disclosure
FIG. 2 illustrates an in-vitro platelet evaluation method according to an implementation of the present disclosure.
FIGS. 3-4 are photographs illustrating the difference between single platelets and aggregated platelets resting on a collagen substrate.

It should be noted that some details of the figures have been simplified and are drawn to facilitate understanding of the present teachings rather than to maintain strict structural accuracy, detail, and scale.

## DETAILED DESCRIPTION

Reference will now be made in detail to exemplary implementations of the present teachings, examples of which are illustrated in the accompanying drawings. Generally, the same reference numbers will be used throughout the drawings to refer to the same or like parts.

The embodiments are described below to provide a more complete understanding of the components, processes, compositions, and apparatuses disclosed herein. Any examples given are intended to be illustrative, and not restrictive. However, it will be apparent to one of ordinary skill in the art that the invention may be practiced without these specific details. In other instances, well-known methods, procedures, and components have not been described in detail so as not to unnecessarily obscure aspects of the embodiments.

Throughout the specification and claims, the following terms take the meanings explicitly associated herein, unless the context clearly dictates otherwise. Phrases such as "in an embodiment," "in certain embodiments," and "in some embodiments" as used herein do not necessarily refer to the same embodiment(s), though they may. Furthermore, the phrases "in another embodiment" and "in some other embodiments" as used herein do not necessarily refer to a different embodiment, although they may.

As used herein, the term "or" is an inclusive operator, and is equivalent to the term "and/or," unless the context clearly dictates otherwise. The term "based on" is not exclusive and allows for being based on additional factors not described, unless the context clearly dictates otherwise. In the specification, the recitation of "at least one of A, B, and C," includes embodiments containing A, B, or C, multiple examples of A, B, or C, or combinations of A/B, A/C, B/C, A/B/B/B/C, A/B/C, etc. In addition, throughout the specification, the meaning of "a," "an," and "the" include plural references. The meaning of "in" includes "in" and "on."

It will also be understood that, although the terms first, second, etc. may be used herein to describe various elements, these elements should not be limited by these terms. These terms are only used to distinguish one element from another. For example, a first object, component, or step could be termed a second object, component, or step, and, similarly, a second object, component, or step could be termed a first object, component, or step, without departing from the scope of the invention. The first object, component, or step, and the second object, component, or step, are both, objects, components, or steps, respectively, but they are not to be considered the same object, component, or step. It will be further understood that the terms "includes," "including," "comprises" and/or "comprising," when used in this specification, specify the presence of stated features, steps, operations, elements, and/or components, but do not preclude the presence or addition of one or more other features, steps, operations, elements, components, and/or groups thereof. Further, as used herein, the term "if" may be construed to mean "when" or "upon" or "in response to determining" or "in response to detecting," depending on the context.

All physical properties that are defined hereinafter are measured at 20° to 25° Celsius unless otherwise specified.

When referring to any numerical range of values herein, such ranges are understood to include each and every number and/or fraction between the stated range minimum and maximum, as well as the endpoints. For example, a range of 0.5-6% would expressly include all intermediate values of, for example, 0.6%, 0.7%, and 0.9%, all the way up to and including 5.95%, 5.97%, and 5.99%, among many others. The same applies to each other numerical property and/or elemental range set forth herein, unless the context clearly dictates otherwise.

Additionally, all numerical values are "about" or "approximately" the indicated value, and take into account experimental error and variations that would be expected by a person having ordinary skill in the art. It should be appreciated that all numerical values and ranges disclosed herein are approximate values and ranges, whether "about" is used in conjunction therewith.

Unless otherwise specified, all percentages and amounts expressed herein and elsewhere in the specification should be understood to refer to percentages by weight. The amounts given are based on the active weight of the material.

As used herein, "gum bleeding" or "bleeding," refers to blood escaping from damaged blood vessels in the circulatory system. For example, as described above, gum bleeding may result from blood escaping from damaged capillaries in the gums surrounding a tooth, and may be a symptom of gingivitis or periodontal disease.

Platelets are a component of blood which react to bleeding from a blood vessel injury by clumping and initiating a blood clot. That is, platelet aggregation is the first step of blood coagulation, which includes the platelet adhesion and formation of a soft aggregate plug.

The effectiveness of an oral care composition or of an active ingredient to prevent, reduce, or stop gum bleeding may be measured in terms of platelet aggregation area and/or platelet rolling velocity. For example, anti-gum bleeding or hemostatic effectiveness may be evidenced by a higher platelet aggregation area and/or a lower platelet rolling velocity.

The inventors have developed a novel in-vitro platelet evaluation system and method to evaluate the effectiveness of oral care compositions or active ingredients on gum bleeding or as hemostatic agents. In particular, a novel flow chamber system was designed to evaluate the growth and stability of platelets aggregation in the presence of specific oral care compositions or active ingredients. In some implementations, the flow chamber system is configured to represent capillary bleeding models and may be used to measure the effect of oral care compositions or active ingredients on platelet aggregation area and/or platelet rolling velocity.

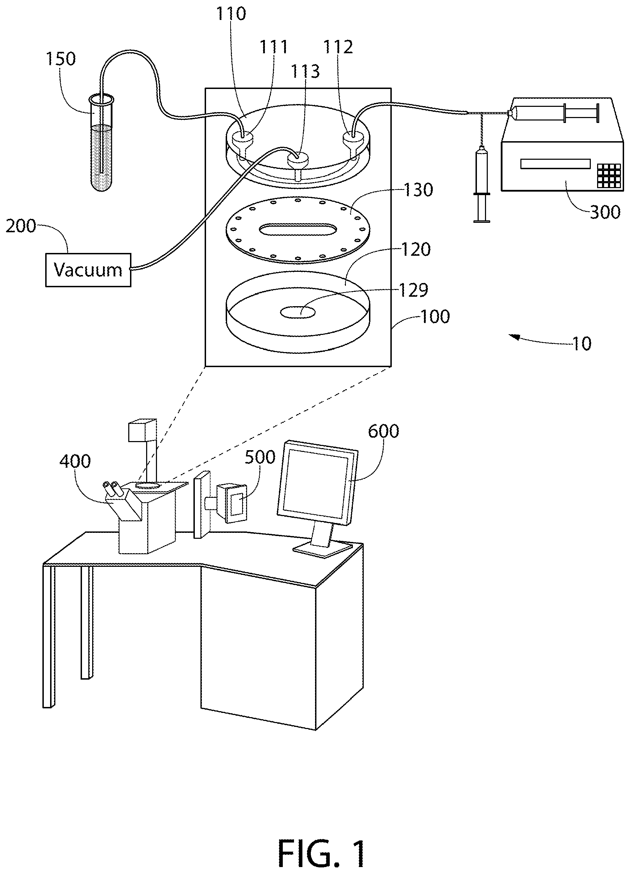

FIG. 1 illustrates an in-vitro platelet evaluation system according to an implementation of the present disclosure. As illustrated in FIG. 1, an in-vitro platelet evaluation system 10 includes a flow chamber 100, a media source 150, a vacuum pump 200, a flow pump 300, a microscope 400, a camera 500, and a computer 600.

The flow chamber 100 may be implemented as a parallel-plate flow chamber 100. For example, the flow chamber 100 may be a circular parallel-plate flow chamber 100 including a base plate 110, a slide plate 120, and a gasket 130 (for example a GlycoTech Flow Chamber 31-001 circular parallel-plate flow chamber available commercially from GlycoTech Corporation, USA).

The base plate 110 may include a flow inlet 111 and a flow outlet 112 through which a test media may be perfused. The base plate 110 may also include a vacuum port 113 through which a vacuum may be applied. In some implementations, the vacuum is configured to hold the base plate 110, gasket 130, and slide plate 120 together and help maintain a uniform channel height. In some implementations, the test media may include a platelet suspension sample and/or a test platelet suspension including suitable amounts of an oral care composition or active ingredient sample for evaluation.

The slide plate 120 may be configured to hold a testing substrate 129. For example, the slide plate 120 may be implemented as a 35mm cell culture petri dish (which may, for example, be made of glass) and configured to hold a collagen substrate.

The gasket 130 is configured to control the height of the flow path for the flow chamber 100. For example, the width or thickness of the gasket 130 may be selected to control a gap height of the flow chamber 100. That is, the gasket thickness may control the channel height. The gasket 130 may be selected according to the chamber type, test sample, and wall shear stress desired.

In one implementation, the channel height may be 0.127 mm, the width may be 2.5 mm, and the length may be 5 mm. The flow chamber may be configured to provide parallel plate dynamic flow with stable viscosity.

The media source 150 is configured to hold the test media that will flow through the flow chamber 100. The media source 150 may be connected to the flow inlet 111 to provide test media to the flow chamber 100. The test media may include platelet suspension samples for testing as a control and/or test platelet suspensions including suitable amounts of an oral care composition or active ingredient for evaluation as hemostatic agents. In some implementations, the flow chamber 100 is maintained at 37°C.

The vacuum pump 200 connects to the vacuum port 113 and is configured to keep a vacuum environment in the flow chamber 100. In some implementations, the vacuum pump 200 also facilitates a stable flow rate for the in-vitro platelet evaluation system 10. For example, the vacuum pump 200 may be configured to expel air and fluidly connect the in-vitro platelet evaluation system 10 to generate a stable flow.

The flow pump 300 is configured to provide a dynamic flow to the system. For example, the flow pump 300 can be implemented as a syringe flow pump 300 connected to the flow outlet 110. Examples of a syringe flow pump 300 are available commercially, for example, the Harvard PHD 22/2000 syringe pump from Harvard Apparatus, Holliston, MA.

The flow chamber 100 may be placed within a microscope 400 (for example an Axio Observer A1, available commercially from Zeiss, Germany) connected to a high speed camera 500 (for example a Mikrotron GmbH MC 1310; available commercially from Norpix Inc., Canada). The microscope 400 and the camera 500 may be connected to a computer 600 to facilitate the observation and recording of the media as it flows and/or is deposited in the flow chamber 100. For example, the computer 600 may include image analysis software for automated image analysis.

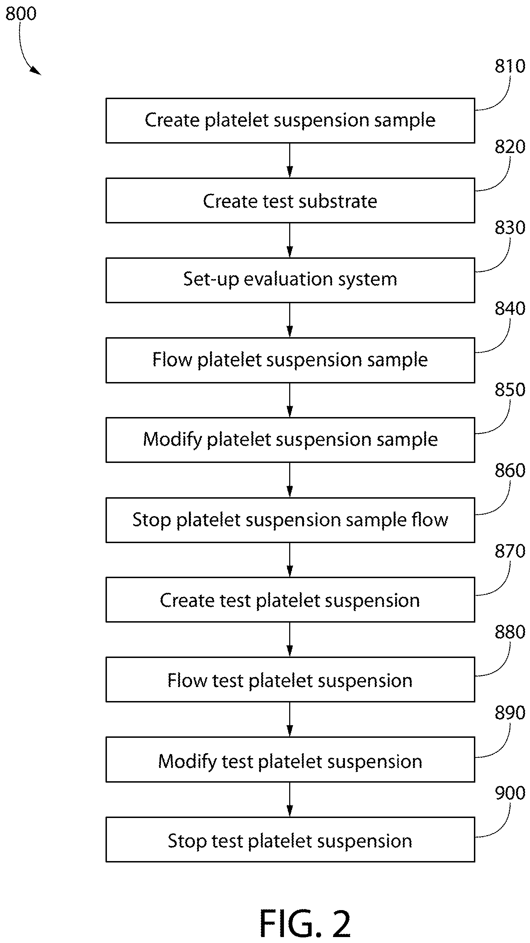

FIG. 2 illustrates an in-vitro platelet evaluation method according to an implementation of the present disclosure. The in-vitro platelet evaluation method 800 may be described with respect to the in-vitro platelet evaluation system 10 of FIG. 1.

The method 800 may begin with the creation of a platelet suspension sample in operation 810. For example, samples of fresh blood may be centrifuged and/or aspirated to separate platelets from the blood, and then, the platelets may be re-suspended in a neutral solution, such as PBS, to create a platelet suspension sample. In some implementations, each platelet suspension sample contains a predetermined number of platelets. In some implementations the platelet suspension sample may also act as a control sample.

In operation 820, a test substrate may be prepared. For example, a collagen substrate may be placed on a slide plate 120 and incubated to prepare the collage substrate to interact with the platelet suspension. While not bound to any particular theory, the inventors believe that during coagulation, platelets adhere to one another and to collagen to form a platelet plug. Accordingly, after infusing the platelet solution into a flow chamber 100, platelets may interact with collagen and bind each other on the collagen substrate in the slide plate 120. Effective coagulants may not only help platelets bind to the collagen substrate in the flow chamber 100 to slow down their rolling velocity, but may also enhance the stable platelet-platelets adhesion, which bring more aggregated platelets to the sample, increasing the total platelet aggregation area.

In operation 830, the evaluation system is set up. For example, the in-vitro platelet evaluation system 10 may be set up: the slide plate 120 (with the collagen substrate placed therein) may be placed together with the gasket 120 and the base plate 110 to form the flow chamber 100. The platelet suspension sample may be placed within the media source 150, and the media source 150 may be connected to the flow inlet 111 of the flow chamber 100. Similarly, the flow outlet 112 may be connected to a syringe flow pump 300, and the vacuum port 113 may be connected to a vacuum pump 200. The flow chamber 100 may then be placed within the microscope 400 and/or the camera 500 (connected to the computer 600) configured to observe, record, and measure the behavior of the platelet suspension sample within the in-vitro platelet evaluation system 10.

In operation 840, the platelet suspension sample is flowed into the flow chamber 110. For example, the flow chamber 100 may be infused with the platelet suspension sample from the media source 150, and the syringe flow pump 300 may be configured to create a flow of the platelet suspension sample at 0.04 mL/min (0.1 dyn/cm$^2$ flow stress) through the flow chamber 100. However, a higher flow rate may be used initially to load the platelet suspension sample into the flow chamber 110. The platelet suspension sample may be initially flowed through the flow chamber 100 for 2 minutes. In some implementations, the flow rate in operation 840 may be in the range 0~0.5 dyn/cm$^2$. In some implementations, the flow chamber 110 is kept at room temp (25° C).

The 0.04 mL/min flow rate is used to generate a 0.1 dyn/cm$^2$ flow stress according to the following volumetric flow rate equation:

$$(1) \; \tau_w = \frac{6\mu Q}{a^2 b}.$$

wherein $\tau_w$ is the wall shear stress in dyn/cm$^2$, $\mu$ is the apparent viscosity of the media (for example, H$_2$O at 37°C = 0.0076P), Q is the volumetric flow rate in l/sec, "a" is the channel height (gasket thickness in cm), and "b" is the channel width (gasket width in cm).

In operation 850, the flow rate is modified and the platelet behavior is recorded. For example, the syringe flow pump 300 may be configured to modify (or maintain) the flow of the platelet suspension sample to 0.04 mL/min through the flow chamber 100. In some implementations, the 0.04 mL/min (0.1 dyn/cm$^2$) flow rate mimics the blood shear stress in oral capillary vessels. In other implementations, the flow rate is modified to less than 0.2 dyn/cm$^2$ to mimic a capillary bleeding model. In yet other implementations, the flow rate is modified to mimic a capillary bleeding model.

The platelet suspension sample may flow through the flow chamber 100 for another 3 minutes, and the camera 500 may be set up to record 2 minutes of the platelet behavior at high speed (50 fps/s). In some implementations, the high speed recording may be used to calculate the platelet rolling velocity and/or platelet aggregation area of the sample. The 2 minute length may be used to simulate an average brushing time, and thus, simulate the period of exposure to the oral care composition or active ingredient being evaluated. However, other video lengths may be used, from less than 1 minute to 2.5 mins and up to 90 minutes, according to the expected period of exposure to the oral care composition or active ingredient being evaluated as hemostatic agents.

The platelet rolling velocity for the platelet suspension sample can then be measured. For example, the rolling distance of each platelet in the platelet suspension sample can be tracked for 30 seconds (from the 90 sec. point to the 120 sec. point) of the high speed recording above. The rolling velocity for the platelet suspension sample is then obtained by averaging all the rolling velocities obtained. In order to facilitate the measurement of platelet rolling velocity, in some implementations, only platelets having a specific mean diameter will be tracked and measure. For example, only platelets having a mean diameter of 2.557 +/- 0.929 $\mu$m are tracked to measure their rolling velocity. In other implementations, a set number of platelets is selected for tracking. For example, 50 platelets having a required mean diameter may be tracked to establish the platelet rolling velocity of the sample.

In some implementations, the computer 600 may be used to perform image analysis on the high speed recording to facilitate measurement of the platelet rolling velocity and/or the platelet aggregation area for the platelet suspension sample. For example, the platelet rolling distance may be captured by a camera 500 operably connected to the microscope 400, and the rolling velocity may be measured in a certain period of time by imaging software (such as Image Pro® Plus 6.0 version, Media Cybernetics), by a computer 600 operably connected to both the camera 500 and the microscope 400.

In operation 860, the platelet suspension sample flow is stopped and the rest volume is measured. For example, the syringe flow pump 300 may be configured to stop the flow of the platelet suspension sample through the flow chamber 100. The rest volume of platelets and/or platelet aggregate area on the collagen substrate is then observed. For example, the total sum area of adhered platelets on the collagen substrate can be measured at the 120 sec. point of the high speed recording above.

For example, the platelet aggregation area may be captured by a camera 500 operably connected to the microscope 400, and the platelet aggregation area may be measured in a certain period of time by imaging software (such as Image Pro® Plus 6.0 version, Media Cybernetics), by a computer 600 operably connected to both the camera 500 and the microscope 400. In some implementations, a representative sample was used to calculate an average platelet aggregation area. For example, the computer may be configured to recognize only platelets in the range of 2~18 $\mu$m$^2$, a traditional range for platelet area, and measure 200 platelets cells at during each period of time.

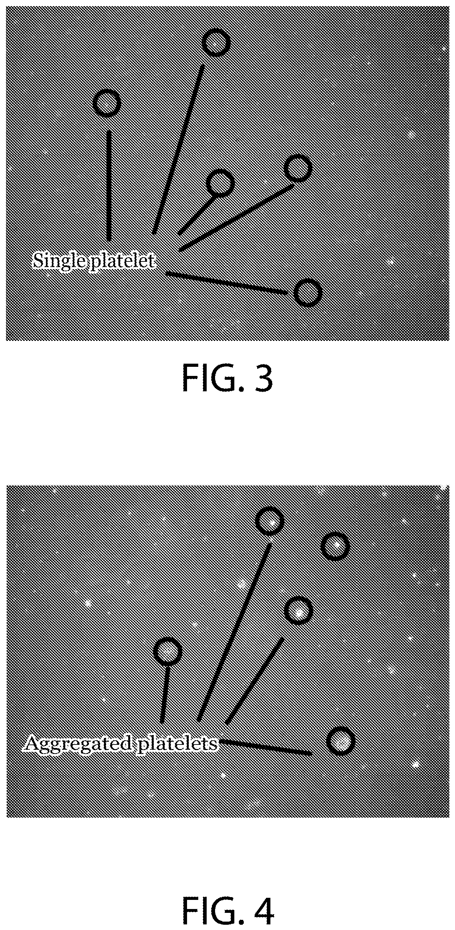

FIGS. 3-4 are photographs illustrating the difference between single platelets and aggregated platelets resting on a collagen substrate, as identified by the computer 600 operably connected to both the camera 500 and the microscope 400.

In operation 870, a test platelet suspension is created. For example, a suitable concentration of an oral care composition or active ingredient to be evaluated is added to the platelet suspension sample in the medium source 150 to create a test platelet suspension. In one implementation, 10ul of an oral composition sample may be added to 1000ul of the platelet suspension sample, that is, at a 1:100 ratio.

In operation 880, the test platelet suspension is flowed into the flow chamber. For example, the flow chamber 100 may be infused with the test platelet suspension from the media source 150, and the syringe flow pump 300 may be configured to create a flow of the test platelet suspension at 0.32 mL/min through the flow chamber 100. The platelet suspension may be initially flowed through the flow chamber 100 for 4 minutes.

In operation 890, the flow rate is changed and the platelet behavior is recorded. For example, the syringe flow pump 300 may be configured to modify the flow of the test platelet suspension to mimic a capillary bleeding model, such as 0.04 mL/min, through the flow chamber 100. The test platelet suspension may flow through the flow chamber 100 for another 3 minutes, and the camera 500 may be set up to record 2 minutes of the platelet behavior at high speed (50 fps/s).

Similarly as above, the platelet rolling velocity for the test platelet suspension can then be measured by tracking and averaging the rolling distance for each platelet in the test platelet suspension for 30 seconds of the high speed recording above.

As described above, the computer 600 may be used to perform image analysis on the high speed recording to facilitate measurement of the platelet rolling velocity and/or the platelet aggregation area for the test platelet suspensions.

In operation 900, the test platelet suspension flow is stopped and the rest volume is measured. For example, the syringe flow pump 300 may be configured to stop the flow of the test platelet suspension through the flow chamber 100. The rest volume of platelets and/or platelet aggregates on the collagen substrate is then observed.

As described above, the platelet aggregation area and platelet rolling velocity were measured for both the platelet suspension sample and the test platelet suspension including the oral care composition or active ingredient samples to be evaluated. For example, the camera 500 was used to record video (2 min. at 50 fps/s) of the platelet behaviors for both types of samples through the system 10.

The platelet rolling velocity was captured by measuring the rolling distance of each platelet during a 30 sec. period (from the 90 sec. point to the 120 sec. point). The rolling velocity for the platelets was then averaged to obtain platelet rolling velocity for the sample. Similarly, the sum area of adhered platelets on the collagen substrate was measured at different time point (30 sec., 60 sec., and 120 sec.) to determine the platelet aggregation area. Because the platelet aggregation area was continually increasing, and because the highest area was found at 120 sec., the measurement at 120 sec. can be used as a standard statistic time point for platelet aggregation area assessment.

## EXAMPLES

Aspects of the present disclosure may be further understood by referring to the following examples. The examples are illustrative, and are not intended to be limiting embodiments thereof.

Table 1 illustrates three oral care compositions. The compositions of Table 1 had the same amount for all ingredients except that Test Composition 1 included both oleanic acid and eugenol, while Test Composition 2 and Test Composition 3 included only oleanic acid or eugenol, respectively.

TABLE 1

| Ingredient | Test Composition #1 | Test Composition #2 | Test Composition #3 |
|---|---|---|---|
| Oleanolic Acid | 0.1 % | 0.1 % | - |
| Eugenol | 0.05% | - | 0.05 % |
| Humectant | 31 % | | |
| Thickener | 1.0 % | | |
| Abrasives | 45% | | |
| Surfactants | 2.55% | | |
| Sodium monofluorophosphate | 1.1 % | | |
| Tetra sodium pyrophosphate | 0.5 % | | |
| Water, flavor, sweetener and minors | q.s | q.s | q.s |

As illustrated in Example 1 below, the compositions of Table 1 were evaluated in terms of platelet aggregation area and platelet rolling velocity using the in-vitro platelet evaluation system and method illustrated in FIG. 1-2. In particular, Table 2 describes platelet aggregation area data for the compositions of Table 1. Table 3 describes platelet rolling velocity data for the compositions of Table 1. As described above, platelet aggregation area and platelet rolling velocity may be used to determine the efficacy of oral care compositions and/or active ingredients to prevent, reduce, or stop gum bleeding.

EXAMPLE 1

Blood platelets were obtained and purified from healthy human donors as follows: 10 mL of Human blood was collected in an anticoagulation tube and centrifuged at 150 g for 15 minutes at room temperature. After that, the upper platelet rich plasma (PRP) layer was aspirated into another centrifuge tube and centrifuged at 900 g for 15 minutes at room temperature. The upper platelet poor plasma (PPP) layer was aspirated, and 1 mL of PBS buffer was added to re-suspend the deposited platelets in the 15 ml centrifuge tube to create a suspended platelet solution. The suspended platelet solution was placed under a microscope (AXIO OBSERVER A1, Zeiss AG, Jena, Germany) and the number of platelets was counted with a haemocytometer. Sample platelet suspension tubes containing $3\text{-}6 \times 10^5$ platelets / mL were then prepared, each with 5 mL of PBS volume.

An in-vitro platelet evaluation was then performed to measure platelet aggregation area and platelet rolling velocity as follows: 20 $\mu$L of 200 $\mu$g/mL collagen was incubated on a 35 mm petri dish (in central 5 mm x 2.5 mm region) overnight (15-18 hours) at 4 °C. The petri dishes were then washed with 1 % BSA PBS buffer 3 times and incubated with 1 % BSA PBS solution for 0.5 hours at room temperature.

A flow chamber system (GLYCOTECH parallel-plate flow chamber, GlycoTech Corp., Gaithersburg, MD) was set up and the platelet suspension was infused with a 0.04 mL/min (1 dyn/cm$^2$) flow for 3 minutes. The flow rate was then changed or maintained to 0.04 mL/min (0.1 dyn/cm$^2$) to reference blood shear stress in oral capillary vessels for 3 minutes, and a high speed camera (50 fps - MIKROTRON MC1310, Mikrotron GmbH Unterschleissheim, Germany) was used to record 2 minutes of the base platelet rolling behavior (control sample). The flow was then stopped and the rest volume of the platelet suspension was observed. 10 $\mu$L of the compositions of Table 1 were then added to the platelet suspension samples. The platelet suspension samples were then infused with a 0.32 mL/min (8 dyn/cm$^2$) flow for 4 minutes (total 1.2 mL suspension). The flow rate was then changed to 0.04 mL/min (0.1 dyn/cm$^2$) for 3 minutes, and a high speed camera (50 fps) was used to record 2 minutes of the platelet rolling behavior after addition of each of the platelet suspension samples including the sample of the compositions of Table 1 (active samples).

The platelet aggregation area and platelet rolling velocity data was analyzed and tracked with image analysis software (IMAGE PRO® PLUS available from Media Cybernetics, Silver Spring, Maryland) and calculated as follows:

Platelet Aggregation Area

The sum area of adhered platelets at different times (30 sec, 60 sec, 120 sec) was measured from the 2 minute high speed recording for each platelet solution sample. The increase in platelet aggregation area for each sample at 120 seconds over the control sample was then calculated using the following formula and averaged over three measurements for each sample:

$$\text{Platelet Aggregation Area (120 seconds)} =$$

$$100 \times \frac{(\text{sum area of platelets - active sample}) - (\text{sum area of platelets - control sample})}{(\text{sum area of platelets - control sample})}$$

Platelet Rolling Velocity

Platelets having a mean diameter of 2.557 +/- 0.929 $\mu$m were selected to measure the average platelet rolling velocity. The rolling velocity for 50 platelets was captured and calculated from the 2 minute high speed recording for each platelet solution sample from the 90 second to the 120 second mark. The reduction in average platelet rolling velocity for each platelet solution over the control sample was then calculated using the following formula and averaged over three measurements for each sample:

$$\text{Platelet Rolling Velocity (90-120 seconds)} =$$

$$100 \times \frac{(\text{rolling velocity - control sample}) - (\text{rolling velocity - active sample})}{(\text{rolling velocity - control sample})}$$

TABLE 2

| | Test Composition #1 | Test Composition #2 | Test Composition #3 |
|---|---|---|---|
| Test | 7.94 | 7.36 | 7.52 |
| Control | 5.93 | 5.99 | 5.92 |
| Average Platelet Aggregation Area Increase over Control | 33.72 % | 22.98 % | 26.94% |

TABLE 3

| | Test Composition #1 | Test Composition #2 | Test Composition #3 |
|---|---|---|---|
| Test | 7.24 | 8.24 | 7.69 |
| Control | 9.89 | 9.72 | 9.89 |
| Average Platelet Rolling Velocity Decrease over Control | 26.76 % | 15.21% | 22.23% |

As illustrated in Tables 2-3, the in-vitro platelet evaluation system and method of the present invention can be used to evaluate the effects on platelet aggregation area and platelet rolling velocity of oral care composition or active ingredient samples. For example, as illustrated in FIGS. 2-3 above, Test Composition 1 displayed an improved increase in average platelet aggregation area and improved decrease in average platelet rolling velocity when compare to Test Compositions 1-2.

The in-vitro platelet evaluation system and method of the present invention can provide quicker evaluation that traditional animal testing or clinical tests. For example, traditional animal testing or clinical testing take weeks to months to perform, while the in-vitro platelet evaluation system and method of the present invention may be performed in less than 1 day, less than 6 hours, less than 4 hours, or about 2 hours or less.

**Claims**

1. An in-vitro platelet evaluation method (800), comprising:

creating (810) a platelet suspension sample;
creating (820) a test substrate, wherein the test substrate comprises collagen;
setting up (830) an evaluation system (10), including placing the test substrate within the evaluation system (10);
flowing (840) the platelet suspension sample through the evaluation system (10);
modifying (850) the platelet suspension sample flow through the evaluation system (10) to mimic a capillary bleeding model flow through the evaluation system (10), wherein the flow rate to mimic a capillary bleeding model through the evaluation system (10) is 0.04 mL/min;
wherein the method (800) further comprises recording (850) a behavior of the platelets in the platelet suspension sample in the evaluation system (10) that is used to determine the platelet aggregation area and platelet rolling velocity for the platelet suspension sample;
stopping (860) the platelet suspension sample flow through the evaluation system (10);
creating (870) a test platelet suspension, wherein creating a test platelet suspension comprises adding a sample of an oral care composition or active agent to the platelet suspension sample to create the test platelet suspension;
flowing (880) the test platelet suspension through the evaluation system (10);
modifying (890) the test platelet suspension flow through the evaluation system (10) to mimic a capillary bleeding model flow through the evaluation system, wherein the flow rate to mimic a capillary bleeding model through the evaluation system (10) is 0.04 mL/min;
wherein the method (800) further comprises recording a behavior of the platelets in the test platelet suspension sample in the evaluation system (10) that is used to determine the platelet aggregation area and platelet rolling velocity for the test platelet suspension sample;
stopping the test platelet suspension flow (900) through the evaluation system (10); and
wherein the evaluation system (10) comprises a vacuum pump (200).

2. The in-vitro platelet evaluation method (800) of claim 1, wherein the evaluation system (10) comprises:

a media source (150) that holds at least one of the platelet suspension sample and the test platelet suspension,
a flow chamber that receives at least one of the platelet suspension sample and the test platelet suspension from the media source,
a flow pump (300) that facilitates flow of at least one of the platelet suspension sample and the test platelet suspension through the flow chamber (100),
a microscope (400) that is configured to evaluate at least one of the platelet suspension sample and the test platelet suspension in the flow chamber (100), and
a camera (500) and a computer (600) that are operably connected to the microscope (400) and that are configured to record an image through the microscope (400) and facilitate evaluation of at least one of the platelet suspension sample and the test platelet suspension in the flow chamber (100), and wherein the vacuum pump (200) is configured to keep the flow chamber (100) in a vacuum environment.

3. The in-vitro platelet evaluation method (800) of claim 2, wherein the flow chamber (100) is a parallel-plate flow chamber comprising:

a base plate (110) configured to control the flow of at least one of the platelet suspension sample and the test platelet suspension through the flow chamber (100),
a slide plate (120) configured to receive at least one of the platelet suspension sample and the test platelet suspension from the media source (150) , and
a gasket (130) configured to control a height of a flow path in the flow chamber,
wherein the base plate (110) comprises:

a flow inlet (111) configured to connect to the media source (150),
a flow outlet (112) configured to connect to the flow pump (300), and
a vacuum port (113) configured to connect to the vacuum pump (200).

4. The in-vitro platelet evaluation method (800) of claim 1, wherein a behavior of the platelets in the platelet suspension sample is recorded for 2 minutes at 50 fps/s.

5. The in-vitro platelet evaluation method (800) of claim 1, wherein a behavior of the platelets in the test platelet suspension is recorded for 2 minutes at 50 fps/s.

6. An in-vitro platelet evaluation method (800), comprising:

creating (810) a platelet suspension sample;
creating (870) a test platelet suspension by adding a test agent to the platelet suspension sample;
flowing (840) the platelet suspension sample through an evaluation system (10) and recording a behavior of platelets in the platelet suspension sample in the evaluation system (10);
flowing (880) the test platelet suspension through the evaluation system (10) and recording a behavior of platelets in the test platelet suspension in the evaluation system (10);
comparing the behavior of platelets in the platelet suspension sample in the evaluation system (10) to the behavior of platelets in the test platelet suspension in the evaluation system (10) to determine a hemostatic efficacy of the test agent;
wherein the flow rate of the platelet suspension sample through the evaluation system (10) and the flow rate of the test platelet suspension through the evaluation system (10) comprises modifying (890) a flow rate to mimic a capillary bleeding model flow through the evaluation system (10),
wherein the flow rate to mimic a capillary bleeding model through the evaluation system (10) is 0.04 mL/min,
wherein comparing the behavior of the platelets comprises comparing an average platelet aggregation area and an average platelet rolling velocity of the platelet suspension sample and the test platelet suspension; and
wherein the evaluation system (10) comprises a vacuum pump (200).

**Patentansprüche**

1. In-vitro-Thrombozyten-Bewertungsverfahren (800), umfassend:

Erzeugen (810) einer Thrombozytensuspensionsprobe;

Erzeugen (820) eines Testsubstrats, wobei das Testsubstrat Kollagen umfasst;

Einrichten (830) eines Bewertungssystems (10), einschließlich des Platzierens des Testsubstrats innerhalb des Bewertungssystems (10);

Durchleiten (840) der Thrombozytensuspensionsprobe durch das Bewertungssystem (10);

Modifizieren (850) des Durchflusses der Thrombozytensuspensionsprobe durch das Bewertungssystem (10), um einen Durchfluss nach einem Kapillarblutungsmodell durch das Bewertungssystem (10) nachzuahmen, wobei die Durchflussrate zur Nachahmung eines Kapillarblutungsmodells durch das Bewertungssystem (10) 0,04 ml/min beträgt;

wobei das Verfahren (800) ferner das Aufzeichnen (850) eines Verhaltens der Thrombozyten in der Thrombozytensuspensionsprobe im Bewertungssystems (10) umfasst, das zur Bestimmung der Thrombozytenaggregationsfläche und der Thrombozytenrollgeschwindigkeit für die Thrombozytensuspensionsprobe verwendet wird;

Stoppen (860) des Durchflusses der Thrombozytensuspensionsprobe durch das Bewertungssystems (10);

Erzeugen (870) einer Test-Thrombozytensuspension, wobei das Erzeugen einer Test-Thrombozytensuspension das Hinzufügen einer Probe einer Mundpflegezusammensetzung oder eines Wirkstoffs zur Thrombozytensuspensionsprobe umfasst, um die Test-Thrombozytensuspension zu erzeugen;

Durchleiten (880) der Test-Thrombozytensuspension durch das Bewertungssystems (10);

Modifizieren (890) des Durchflusses der Test-Thrombozytensuspension durch das Bewertungssystems (10), um einen Durchfluss nach einem Kapillarblutungsmodell durch das Bewertungssystems nachzuahmen, wobei die Durchflussrate zur Nachahmung eines Kapillarblutungsmodells durch das Bewertungssystems (10) 0,04 ml/min beträgt;

wobei das Verfahren (800) ferner das Aufzeichnen eines Verhaltens der Thrombozyten in der Test-Thrombozytensuspensionsprobe im Bewertungssystem (10) umfasst, das zur Bestimmung der Thrombozytenaggregationsfläche und der Thrombozytenrollgeschwindigkeit für die Test-Thrombozytensuspensionsprobe verwendet wird;

Stoppen des Durchflusses der Test-Thrombozytensuspension (900) durch das Bewertungssystems (10); und

wobei das Bewertungssystems (10) eine Vakuumpumpe (200) umfasst.

2.  In-vitro-Thrombozyten-Bewertungsverfahren (800) nach Anspruch 1, wobei das Bewertungssystems (10) umfasst:

eine Medienquelle (150), die mindestens eine der Thrombozytensuspensionsprobe und der Test-Thrombozytensuspension enthält,

eine Durchflusskammer, die mindestens eine der Thrombozytensuspensionsprobe und der Test-Thrombozytensuspension von der Medienquelle aufnimmt,

eine Durchflusspumpe (300), die den Durchfluss mindestens einer der Thrombozytensuspensionsprobe und der Test-Thrombozytensuspension durch die Durchflusskammer (100) unterstützt,

ein Mikroskop (400), das dazu ausgebildet ist, mindestens eine der Thrombozytensuspensionsprobe und der Test-Thrombozytensuspension in der Durchflusskammer (100) zu bewerten, und

eine Kamera (500) und einen Computer (600), die funktionsfähig mit dem Mikroskop (400) verbunden sind und dazu ausgebildet sind, ein Bild durch das Mikroskop (400) aufzunehmen und die Auswertung mindestens einer der Thrombozytensuspensionsprobe und der Test-Thrombozytensuspension in der Durchflusskammer (100) zu unterstützen, und wobei die Vakuumpumpe (200) dazu ausgebildet ist, die Durchflusskammer (100) in einer Vakuumumgebung zu halten.

3.  In-vitro-Thrombozyten-Bewertungsverfahren (800) nach Anspruch 2, wobei die Durchflusskammer (100) eine Parallelplatten-Durchflusskammer ist, umfassend:

eine Grundplatte (110), die dazu ausgebildet ist, den Durchfluss mindestens einer der Thrombozytensuspensionsprobe und der Test-Thrombozytensuspension durch die Durchflusskammer (100) zu steuern,

eine Gleitplatte (120), die dazu ausgebildet ist, mindestens eine der Thrombozytensuspensionsprobe und der Test-Thrombozytensuspension von der Medienquelle (150) aufzunehmen, und

eine Dichtung (130), die dazu ausgebildet ist, eine Höhe eines Durchflusswegs in der Durchflusskammer zu steuern,

wobei die Grundplatte (110) umfasst:

einen Durchflusseinlass (111), der dazu ausgebildet ist, sich mit der Medienquelle (150) zu verbinden,

einen Durchflussauslass (112), der dazu ausgebildet ist, sich mit der Durchflusspumpe (300) zu verbinden, und

einen Vakuumanschluss (113), der dazu ausgebildet ist, sich mit der Vakuumpumpe (200) zu verbinden.

4. In-vitro-Thrombozyten-Bewertungsverfahren (800) nach Anspruch 1, wobei ein Verhalten der Thrombozyten in der Thrombozytensuspensionsprobe 2 Minuten lang mit 50 Bildern pro Sekunde aufgezeichnet wird.

5. In-vitro-Thrombozyten-Bewertungsverfahren (800) nach Anspruch 1, wobei ein Verhalten der Thrombozyten in der Test-Thrombozytensuspension 2 Minuten lang mit 50 Bildern pro Sekunde aufgezeichnet wird.

6. In-vitro-Thrombozyten-Bewertungsverfahren (800), umfassend:

Erzeugen (810) einer Thrombozytensuspensionsprobe;
Erzeugen (870) einer Test-Thrombozytensuspension durch Zugabe eines Testwirkstoffs zu der Thrombozytensuspensionsprobe;
Durchleiten (840) der Thrombozytensuspensionsprobe durch ein Bewertungssystem (10) und Aufzeichnen eines Verhaltens der Thrombozyten in der Thrombozytensuspensionsprobe im Bewertungssystem (10);
Durchleiten (880) der Test-Thrombozytensuspension durch das Bewertungssystem (10) und Aufzeichnen eines Verhaltens der Thrombozyten in der Test-Thrombozytensuspension im Bewertungssystem (10);
Vergleichen des Verhaltens der Thrombozyten in der Thrombozytensuspensionsprobe im Bewertungssystem (10) mit dem Verhalten der Thrombozyten in der Test-Thrombozytensuspension im Bewertungssystem (10), um eine hämostatische Wirksamkeit des Testwirkstoffs zu bestimmen;
wobei die Durchflussrate der Thrombozytensuspensionsprobe durch das Bewertungssystem (10) und die Durchflussrate der Test-Thrombozytensuspension durch das Bewertungssystem (10) das Modifizieren (890) einer Durchflussrate umfassen, um einen Durchfluss nach einem Kapillarblutungsmodell durch das Bewertungssystem (10) nachzuahmen,
wobei die Durchflussrate zur Nachahmung eines Kapillarblutungsmodells durch das Bewertungssystem (10) 0,04 ml/min beträgt,
wobei der Vergleich des Verhaltens der Thrombozyten das Vergleichen einer durchschnittlichen Thrombozytenaggregationsfläche und einer durchschnittlichen Thrombozytenrollgeschwindigkeit der Thrombozytensuspensionsprobe und der Test-Thrombozytensuspension umfasst; und
wobei das Bewertungssystem (10) eine Vakuumpumpe (200) umfasst.


## Revendications

1. Procédé d'évaluation in vitro de plaquettes (800), comprenant :

la création (810) d'un échantillon de suspension plaquettaire ;
la création (810) d'un substrat de test, dans lequel le substrat de test comprend du collagène ;
la mise en place (830) d'un système d'évaluation (10), comprenant le placement du substrat de test à l'intérieur du système d'évaluation (10) ;
l'écoulement (840) de l'échantillon de suspension plaquettaire à travers le système d'évaluation (10) ;
la modification (850) du flux d'échantillon de suspension plaquettaire à travers le système d'évaluation (10) pour imiter un flux de modèle de saignement capillaire à travers le système d'évaluation (10), dans lequel le débit pour imiter un modèle de saignement capillaire à travers le système d'évaluation (10) est de 0,04 ml/min ;
dans lequel le procédé (800) comprend en outre l'enregistrement (850) d'un comportement des plaquettes dans l'échantillon de suspension plaquettaire dans le système d'évaluation (10) qui est utilisé pour déterminer la surface d'agrégation plaquettaire et la vitesse de roulement des plaquettes pour l'échantillon de suspension plaquettaire ;
l'arrêt (860) du flux d'échantillon de suspension plaquettaire à travers le système d'évaluation (10) ;
la création (870) d'une suspension plaquettaire de test, dans lequel la création d'une suspension plaquettaire de test comprend l'ajout d'un échantillon d'une composition de soins bucco-dentaires ou d'un principe actif à l'échantillon de suspension plaquettaire afin de créer la suspension plaquettaire de test ;
l'écoulement (880) de la suspension plaquettaire de test à travers le système d'évaluation (10) ;
la modification (890) du flux de suspension plaquettaire de test à travers le système d'évaluation (10) pour imiter un flux de modèle de saignement capillaire à travers le système d'évaluation, dans lequel le débit pour imiter un modèle de saignement capillaire à travers le système d'évaluation (10) est de 0,04 ml/min ;
dans lequel le procédé (800) comprend en outre l'enregistrement d'un comportement des plaquettes dans l'échantillon de suspension plaquettaire de test dans le système d'évaluation (10) qui est utilisé pour déterminer la surface d'agrégation plaquettaire et la vitesse de roulement des plaquettes pour l'échantillon de suspension plaquettaire de test ;

l'arrêt du flux de la suspension plaquettaire de test (900) à travers le système d'évaluation (10) ; et

dans lequel le système d'évaluation (10) comprend une pompe à vide (200).

2. Procédé d'évaluation in vitro de plaquettes (800) selon la revendication 1, dans lequel le système d'évaluation (10) comprend :

une source de milieu (150) qui contient au moins l'un de l'échantillon de suspension plaquettaire et de la suspension plaquettaire de test,

une chambre d'écoulement qui reçoit au moins l'un de l'échantillon de suspension plaquettaire et de la suspension plaquettaire de test provenant de la source de milieu,

une pompe de circulation (300) qui facilite l'écoulement d'au moins l'un de l'échantillon de suspension plaquettaire et de la suspension plaquettaire de test à travers la chambre d'écoulement (100),

un microscope (400) qui est configuré pour évaluer au moins l'un de l'échantillon de suspension plaquettaire et de la suspension plaquettaire de test dans la chambre d'écoulement (100), et

une caméra (500) et un ordinateur (600) qui sont connectés de manière fonctionnelle au microscope (400) et qui sont configurés pour enregistrer une image à travers le microscope (400) et faciliter l'évaluation d'au moins l'un de l'échantillon de suspension plaquettaire et de la suspension plaquettaire de test dans la chambre d'écoulement (100), et dans lequel la pompe à vide (200) est configurée pour maintenir la chambre d'écoulement (100) dans un environnement sous vide.

3. Procédé d'évaluation in vitro de plaquettes (800) selon la revendication 2, dans lequel la chambre d'écoulement (100) est une chambre d'écoulement à plaques parallèles comprenant :

une plaque de base (110) configurée pour réguler l'écoulement d'au moins l'un de l'échantillon de suspension plaquettaire et de la suspension plaquettaire de test à travers la chambre d'écoulement (100),

une plaque coulissante (120) configurée pour recevoir au moins l'un de l'échantillon de suspension plaquettaire et de la suspension plaquettaire de test provenant de la source de milieu (150), et

un joint (130) configuré pour commander une hauteur d'un trajet d'écoulement dans la chambre d'écoulement, dans lequel la plaque de base (110) comprend :

une entrée d'écoulement (111) configurée pour se raccorder à la source de milieu (150),

une sortie d'écoulement (112) configurée pour se raccorder à la pompe de circulation (300), et

un orifice de vide (113) configuré pour se raccorder à la pompe à vide (200).

4. Procédé d'évaluation in vitro de plaquettes (800) selon la revendication 1, dans lequel un comportement des plaquettes dans l'échantillon de suspension plaquettaire est enregistré pendant 2 minutes à une cadence de 50 images par seconde.

5. Procédé d'évaluation in vitro de plaquettes (800) selon la revendication 1, dans lequel un comportement des plaquettes dans la suspension plaquettaire de test est enregistré pendant 2 minutes à une cadence de 50 images par seconde.

6. Procédé d'évaluation in vitro de plaquettes (800), comprenant :

la création (810) d'un échantillon de suspension plaquettaire ;

la création (870) d'une suspension plaquettaire de test par ajout d'un agent de test à l'échantillon de suspension plaquettaire ;

l'écoulement (840) de l'échantillon de suspension plaquettaire à travers un système d'évaluation (10) et l'enregistrement d'un comportement des plaquettes dans l'échantillon de suspension plaquettaire dans le système d'évaluation (10) ;

l'écoulement (880) de la suspension plaquettaire de test à travers le système d'évaluation (10) et l'enregistrement d'un comportement des plaquettes dans la suspension plaquettaire de test dans le système d'évaluation (10) ;

la comparaison du comportement des plaquettes dans l'échantillon de suspension plaquettaire dans le système d'évaluation (10) au comportement des plaquettes dans la suspension plaquettaire de test dans le système d'évaluation (10) afin de déterminer une efficacité hémostatique de l'agent de test ;

dans lequel le débit de l'échantillon de suspension plaquettaire à travers le système d'évaluation (10) et le débit de la suspension plaquettaire de test à travers le système d'évaluation (10) comprennent la modification (890) d'un débit pour imiter un écoulement de modèle de saignement capillaire à travers le système d'évaluation (10), dans lequel le débit pour imiter un modèle de saignement capillaire à travers le système d'évaluation (10) est de 0,04 ml/min,

dans lequel la comparaison du comportement des plaquettes comprend la comparaison d'une surface d'agrégation plaquettaire moyenne et d'une vitesse de roulement plaquettaire moyenne de l'échantillon de suspension plaquettaire et de la suspension plaquettaire de test ; et

dans lequel le système d'évaluation (10) comprend une pompe à vide (200).

FIG. 1

800

810

Create platelet suspension sample

820

Create test substrate

830

Set-up evaluation system

840

Flow platelet suspension sample

850

Modify platelet suspension sample

860

Stop platelet suspension sample flow

870

Create test platelet suspension

880

Flow test platelet suspension

890

Modify test platelet suspension

900

Stop test platelet suspension

FIG. 2

FIG. 3

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110045993 A1

- US 20140255961 A1

**Non-patent literature cited in the description**

- **MENG Q.** Anti-bacterial toothpaste comprises anti-inflammatory hemostatic component, anti-allergic and analgesic component and base component. Clarivate Analytics, 15 March 2017, vol. 2017